(19) Europäisches Patentamt
European Patent Office
Office européen des brevets

(11) Publication number: 0 008 174
B1

(12) EUROPEAN PATENT SPECIFICATION

(45) Date of publication of patent specification: 27.11.85

(21) Application number: 79301469.7

(22) Date of filing: 24.07.79

(51) Int. Cl.$^4$: C 07 C 51/41, C 07 C 53/126, C 07 C 59/265, C 07 C 101/26, C 07 C 65/10, C 07 C 149/20, C 07 C 159/00

# (54) Method of making an organic metal salt or complex.

(30) Priority: 11.08.78 US 932945

(43) Date of publication of application: 20.02.80 Bulletin 80/04

(45) Publication of the grant of the patent: 27.11.85 Bulletin 85/48

(84) Designated Contracting States: AT BE CH DE FR GB IT NL SE

(56) References cited:
DE-C- 968 843
FR-A-1 210 496
GB-A- 928 624
GB-A- 950 574
GB-A-1 053 805
GB-A-1 210 773
US-A-3 476 786
US-A-4 055 655

(73) Proprietor: NATIONAL RESEARCH LABORATORIES
3567 Blue Rock Road
Cincinnati, Ohio 45239 (US)

(72) Inventor: Maurer, Gerald L.
6389 Gray Road
Fairfield, Ohio (US)
Inventor: Stefanini, Virginia E.
4090 Sharon Park Lane
Cincinnati, Ohio (US)

(74) Representative: Allen, Oliver John Richard et al
Lloyd Wise, Tregear & Co. Norman House
105-109 Strand
London, WC2R 0AE (GB)

EP 0 008 174 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of the invention

Heavy metal salts or complexes of organic acids have commonly been prepared by reacting a heavy metal chloride or sulfate with an alkali metal salt of the acid. In these reactions, the usual by-product salts such as NaCl, $Na_2SO_4$, KCl, LiCl and $K_2SO_4$ are highly soluble in aqueous media. In the formation of metal complexes, it has been proposed to remove such by-product salts by a technique of extraction as, for example, disclosed in U.S. Patent 4,055,655. However, such extraction techniques become difficult or even prohibitively expensive. The need for synthesizing second salt-free aqueous and solid preparations of heavy metal salts or chelates is high-lighted by a brief consideration of the end uses of such salts or chelates. For instance, in the case of employing such complexes as antimicrobial agents, the mentioned common second salts are all potentially irritating to normal skin, and markedly irritating to denuded or injured tissue. In industrial applications, such common salts are found to be corrosive to the metals in machinery and work pieces. Thus, a method for the production of metal salts or complexes of organic acids which are second salt-free would be highly desirable, particularly when such complexes are used in any application in which such salts may be potentially harmful or unwanted.

The classic heavy metal donors which are employed in prior art techniques are heavy metal chlorides or sulfates. These classic salts provide lower percentages by weight of the heavy metal ion in the salt in comparison, for example, to the corresponding heavy metal carbonates or hydroxides. For instance, the percent of copper in $CuCl_2 \cdot 2H_2O$ and $CuSO_4 \cdot 5H_2O$ is approximately 37 and 25%, respectively. In contrast, the percent of copper in copper hydroxide is approximately 80%. However, the copper hydroxide as a possible form of heavy metal donor, which potentially provides a more efficient source of heavy metal ion, is relatively inert.

Prior art processes have been developed to provide methods of preparing heavy metal salts which do not involve the double reaction and formation of a second salt. United States Patent Specification No. 3 476 786 discloses finely divided water insoluble metallic salts of higher fatty acids may be produced by grinding solid fatty acids with particular solid metals, metal oxides, metal hydroxides, metal carbonates or mixtures thereof in the presence of a catalyst and in the absence of water. A typical example employs solid calcium hydroxide and solid stearic acid in the presence of ammonium carbonate as the catalyst. British Patent Specification No. 1 053 805 discloses a process for producing dry or substantially dry finely divided alkaline earth or heavy metal salts in admixture with carriers comprising reacting an acid and at least one of an oxide, hydroxide and/or carbonate of a metal in finely divided form and in the presence of an excess by weight of finely divided inorganic carrier material in respect of the reactants which is substantially inert under the conditions of the reaction, the reaction being effected by heating and without the addition of water. British Patent Specification No. 950 574 discloses a method of preparing a metal soap comprising the steps of forming an aqueous suspension of an oxide or hydroxide of a metal capable of forming a basic metal carbonate, reacting such suspension with carbon dioxide while stirring vigorously to form a suspension of the basic metal carbonate, forming an aqueous emulsion of a liquid or molten fatty acid having 6 or more carbon atoms in the presence of ammonia and adding the emulsion to the suspension to form the metal soap. No gas is generated during the reaction of the basic metal carbonate with the acid to form the metal soap.

In accordance with the present invention there is provided a method of preparing a salt or complex of an organic ligand which is a carboxylic acid or salt thereof and a metal selected from zinc, nickel, chromium, bismuth, mercury, silver, copper, magnesium, aluminium and cobalt, in which a hydroxide of such a metal is reacted with said organic ligand in an aqueous medium, characterised in that the reaction is conducted in the presence of an effective catalytic amount of gaseous $CO_2$, which gaseous catalyst may be added to the reaction mixture itself or may be generated from precursors thereof present in the reaction mixture or added to the reaction mixture during the reaction, for example from carbonic acid, bicarbonates or carbonates.

The invention provides a method of making an organic metal salt or complex in a very efficient manner. According to the principles of this invention, the metal complexes or salts may be synthesised in a second salt-free aqueous or solid state. Furthermore, the invention enables the utilization of highly efficient metal donors in the reaction with carboxylic acids. Thus, this invention eliminates the need for utilization of classic heavy metal donors, such as the heavy metal chlorides or sulphates, which provide highly soluble and undesirable by-products in industrial or pharmaceutical applications as developed above. In addition, the method employs metal hydroxides which heretofore have been considered relatively inert or have required special reaction conditions, e.g. absence of water. The metal hydroxides used according to the method of the invention are also more efficient due to their higher percentage of heavy metal ion compared to sulphates, chlorides and other common donors.

Another feature of this invention enables the use of efficient naturally-occurring metal donors. For instance whereas a most efficient donor would be a heavy metal carbonate, heavy metal carbonates are frequently unavailable or prohibitively expensive. It has been found that basic metal carbonates having a general formula

$$xMCO_3 \cdot yM(OH)_2 \cdot zH_2O$$

as commonly found in nature, offer an excellent source of starting material for the inventive reaction, thereby offering significant economies. Such basic metal carbonates offer two efficient sources of metal ion, the metal carbonate and hydroxide. For example, the copper carbonate salt in malachite has been found to provide an effective form of $CO_2$ which drives the heavy metal hydroxide to react with an organic ligand, such as citric acid.

According to the techniques herein disclosed, heavy metal organic complexes or salts can be prepared in pure solution or solid forms. Such pure forms enable the wide utilization of metal complexes or salts, free from corrosion or other problems in industrial applications. Furthermore, purity may be obtained in solution form or reaction products may be isolated by direct crystallisation to a pure solid state.

Another significant aspect of this invention is the provision of means or method for retaining a form of $CO_2$ in the reaction medium such that the reaction may be driven to completion. On one hand, the reaction may be conducted in a sealed reaction vessel or environment for retaining the $CO_2$ where it might otherwise escape. In another form, the reaction has been found capable of control with a buffering agent. By the employment of a buffering agent, the pH is controlled, thereby providing retained $CO_2$ to drive the reaction to completion.

The invention will be better understood with reference to chemical mechanisms believed to be involved. It is to be understood, however, that the empirical results stand on their own and applicants do not herein wish to be limited to specific mechanisms or theories. Still, unique features of this invention and the unexpected results achieved may be elucidated. Reference is first made to the utilization of basic carbonate salts of the metals, represented by the formula

$$xMCO_3 \cdot yM(OH)_2 \cdot zH_2O,$$

as the metal donors for the preparation of chelates with polyfunctional organic ligands, e.g., citrate. In such a typical reaction, the metal carbonate is believed to react to form a bicarbonates ion, which undergoes an initial reaction onto the relatively unreactive $M(OH)_2$ group, apparently to form a more reactive $M(HCO_3)_2$ molecule which then dissolves to form a free metallic cation able to react with the organic ligand such as citrate. The following reaction sequence demonstrates the case of basic copper carbonate and a polyfunctional organic ligand, represented by the letter L.

$$(1) \quad CuCO_3 \overset{H^+}{\rightleftarrows} Cu^{++}+H_2CO_3 \rightleftarrows H_2O+CO_2(\uparrow)$$

The rate of this reaction, and the resultant loss of $CO_2$, is controlled by a citrate buffer maintaining a mildly acidic pH in accordance with the most preferred method of this invention. Where the reaction is not contained, the carbonate lost as $CO_2$ must be replaced for utilization later in the reaction; therefore, a slower reaction rate for this step leads to amore efficient synthesis.

$$(2) \quad Cu^{++}+L^{=} \rightleftarrows CuL^-$$

This reaction, forming the 1:1 Cu:ligand chelate, is spontaneous under the pH conditions of the synthesis.

$$(3) \quad CO_2 \overset{OH^-}{\rightleftarrows} HCO_3^- \rightleftarrows H^+ + CO_3^{=}$$

$$(4) \quad Cu(OH)_2+2HCO_3^- \rightleftarrows Cu(HCO_3)_2+2OH^-$$

The presumed existence of this addition product is supported by the formation, during the reaction, of a poorly soluble green-black material which gradually disappears. The evential completion of this reaction depends upon the added bicarbonate ion, which replenishes the carbonate lost as $CO_2$ gas.

$$(5) \quad Cu(HCO_3)_2 \rightleftarrows Cu^{++}+2HCO_3^-$$

The equilibrium for this reaction lies to the reactant side. Therefore, this reaction is probably the rate-limiting step of the synthesis. The reaction rate of this step is enhanced by the exhaustion of the product, $Cu^{++}$, as shown in Step 2 above, in the subsequent formation of the 1:1 complex.

$$(6) \quad HCO_3^- \rightleftarrows CO_2(\uparrow)+OH^-$$

This equilibrium reaction, which proceeds slowly to the right due to the slower rate of loss of the carbon dioxide in an only slightly acidic medium, is responsible for the eventual exhaustion of the carbonate counter ion from the chelate preparation. The overall reaction is, then,

$$Cu^{++}+L^{=} \rightleftarrows CuL$$

In a preferred form of the method, the organic Lewis acid is prepared in an acidic solution. In the case of strongly acidic organic ligands, a buffer of pH 3.5—4.5 can be employed by mixing appropriate amounts of free carboxylic acid and carboxylic acid salt. In this pH range, the dissolution of a metal carbonate occurs quite slowly, and with little generation of carbon dioxide gas. Retention of carbon dioxide either as a soluble gas, or as a carbonate and/or bicarbonate enables the reaction to proceed slowly. Furthermore, it is desired to employ the most concentrated solutions practical with respect to solubility of reactants and reaction product. The more concentrated solution allows for enhanced reaction rate in Step 4 of the above reaction mechanism scheme, since a greater concentration of reactants allows for more

frequent contact. The basic carbonate salt is added slowly to the carboxylic acid solution to minimize foaming and resultant loss of the carbonate species. In an uncontained system some carbonate is lost, nonetheless, and it is usually replaced as bicarbonate ion, the form necessary for reaction in addition Step 4 above. $CO_2$ in the form of either sodium carbonate, dry ice, liquid $CO_2$ or dissolved carbonate, is also adequate because of the usual equilibrium existing among the various forms. Because of the buffering capacity of a large number of carboxylic acid ligands, adjustment of the pH during the reaction is rarely necessary. After the reaction is complete, the pH may be adjusted for storage or the solid form of the product may be isolated by direct crystallization.

The metal ions may be of a monovalent or polyvalent nature, more specifically monovalent.

The metal ions are selected from zinc, nickel, chromium, bismuth, mercury, silver, cobalt, magnesium, copper and aluminum. The end use or industrial application will dictate the choice of metal ion. In the case of antimicrobial agents, complexes of heavier metals are considered more toxic that those of lighter metals, for instance.

The term "organic ligand" refers to molecules or ions that can combine with another molecule or ion by forming a covalent chemical bond with two electrons from the second molecule or ion. Such a molecule or ion is thus an electron acceptor. Organic ligands for use in the invention may either be of a monofunctional or polyfunctional type and are organic carboxylic acids, including monocarboxylic as well as dicarboxylic acids, or salts thereof characterised by the formula $(RCXX)_nM$ wherein RCXX is either a carboxylate or thiocarboxylate group of an aliphatic or aromatic mono or polyfunctional acid; R is a hydrocarbon or substituted hydrocarbon radical; X is oxygen or sulfur; n is an integral number usually from 1 to 3 and M is a mono or polyvalent metal. Aromatic carboxylates of the phthalic, benzoic or naphthoic type where R is aryl, diaryl or substituted aryl may be employed such as calcium benzoate. Illustrative of the carboxylic acids are palmitic acid, stearic acid, myristic acid, oleic acid, linoleic acid, behenic acid, hexatriacontanoic acid, tetrapropylene-substituted glutaric acid, succinic acid, octadecyl-substituted adipic acid, chlorostearic acid, 9-methylstearic acid, dichlorostearic acid, stearyl-benzoic acid, naphthoic acid, dilauryl-decahydronaphthalene carboxylic acid, didodecyl-tetralin carboxylic acid, dioctyl-cyclohexane carboxylic acid, mixtures of these acids, their alkali and alkaline earth metal salts and/or their anhydrides. An especially suitable group of aliphatic fatty acids includes the saturated and unsaturated higher fatty acids containing from 12 to about 30 carbon atoms. Illustrative of these acids are lauric acid, palmitic acid, oleic acid, linoleic acid, oleo-stearic acid, stearic acid, myristic acid and undecalinic acid, alpha-chlorostearic acid and alpha-nitrolauric acid. The organic acids may contain non-hydrocarbon substituents such as halo, nitro, alkoxy and hydroxyl.

A particularly useful class of metal complexes made according to the method of this invention are those where the metal hydroxide is reacted with a polyfunctional organic ligand. In the Examples which follow, citric acid, mercaptosuccinic acid, ethylenediamine tetraacetic acid and others were used. However, other polyfunctional organic ligands of the group of carboxylic acids may be substituted for such acids specifically exemplified by the operating Examples which follow. Included among other polyfunctional ligands are the broader class of alpha or beta hydroxy polycarboxylic acids represented by citric acid. Other polyfunctional ligands for use in the invention include aminopolycarboxylic, sulfhydropolycarboxylic and phosphinolpolycarboxylic acids. Metal complexes formed with such ligands may be of the 1:1 type or wherein the ratio of metal ion with respect to the polyfunctional ligand varies, as also represented by the dicupric edetate and dicobaltous edetate of the Examples. Such metal complexes of polyfunctional organic ligands have been further disclosed in U.S. A—4,055,655 at Columns 5 and 6, and such disclosures are incorporated herein by reference.

The following examples illustrate various embodiments of this invention.

Example 1

Disodium monocopper(II) citrate complex by basic copper carbonate-citric acid-sodium bicarbonate method

Ingredients:

65 ml water

61 g citric acid, anhydrous

35 g basic copper carbonate $[CuCO_3 \cdot Cu(OH)_2 \cdot H_2O]$

60 g sodium bicarbonate ($NaHCO_3$)

The citric acid was dissolved in the water. The basic copper carbonate was added with stirring and dispersed well. This mixture was allowed to react for approximately 10 minutes or until the foam ($CO_2$ generation) subsided. Sodium bicarbonate was added slowly with gentle mixing until the pH was between 5.5 and 6.0. The solution was mixed until a black granular precipitate was no longer visible $[Cu(HCO_3)_2]$. The remainder of the sodium bicarbonate was added slowly with gentle stirring to adjust the pH to 7.0 for storage. The soluble copper chelate was thus prepared free from a second salt.

Example 2

Disodium monocopper(II) citrate complex by basic copper carbonate-sodium citrate-dry ice method

Ingredients:

75 ml 2.6 *M* aqueous trisodium citrate dihydrate adjusted to pH 5.0 with 2.6 *M* aqueous citric acid
47.8 g basic copper carbonate $[CuCO_3 \cdot Cu(OH)_2 \cdot H_2O]$
ca. 80 g dry ice ($CO_2$) pellets
NaOH flakes

The basic copper carbonate was added slowly to the citrate solution with rapid mixing to effect good dispersion. This mixture was allowed to react for approximately 10 minutes, or until the foam ($CO_2$ generation) subsided. The dry ice was added slowly with constant stirring, over a 10 minute period. The solution was mixed vigorously for approximately 1 hour or until a granular black precipitate $[Cu(HCO_3)_2]$ was no longer visible. For storage of the soluble copper chelate, the pH was adjusted to between 6.5 and 7.0 with NaOH.

Example 3
Disodium monocopper(II) citrate complex by basic copper carbonate-citric acid-dry ice method

Ingredients:

50 ml water
61 g citric acid, anhydrous
35 g basic copper carbonate $[CuCO_3 \cdot Cu(OH)_2 \cdot H_2O]$
60 g dry ice pellets ($CO_2$)
47 g NaOH flakes

The citric acid was dissolved in the water. The basic copper carbonate was added to effect homogeneous dispersion. The mixture was allowed to react for approximately 10 minutes, or until the foam ($CO_2$ generation) subsides. The dry ice was added slowly with constant stirring. The material was allowed to mix until a black granular precipitate was no longer seen $[Cu(HCO_3)_2]$. NaOH flakes were added slowly so that heat generation did not become excessive. The pH of the resultant solution was approximately 7.0, ideal for storage.

Example 4
Copper 3,3′-thiodipropionate by basic copper carbonate-3,3′-thiodipropionic acid-sodium bicarbonate method

Ingredients:

100 ml water
1.2 mg basic copper carbonate
1.78 g 3,3′-thiodipropionic acid, anhydrous
ca. 0.1 g sodium bicarbonate, anhydrous

The 3″-thiodipropionic acid was dissolved in the water. The basic copper carbonate was added slowly with stirring. Gradual evolution of bubbles occurred after enough of the metal salt had been added to raise the pH to ca. 3.2. At this point, the insoluble material began to disappear more rapidly. After all of the bubbling had stopped, the liquid was stirred for approximately 15 minutes, and then the sodium bicarbonate was added slowly, with ample reaction time before any further addition. The reaction was complete when a clear blue-green solution was obtained.

Example 5
Cobaltous mercaptosuccinate by basic cobaltous carbonate-mercaptosuccinic acid-sodium bicarbonate method

Ingredients:

30 ml hot water
1.03 g basic cobaltous carbonate
1.50 g mercaptosuccinic acid
0.2 g sodium bicarbonate
concentrated aqueous NaOH solution

The acid was dissolved in the water, and the cobaltous carbonate was added slowly with vigorous stirring. Gradual development of a rust-brown color was noticeable followed by a deep brown coloration of the supernatant liquid. After all of the cobaltous carbonate was added, the mixture was allowed to react for 30—40 minutes, and then the sodium bicarbonate was added very slowly to minimize foaming. When all of the insoluble material was gone, the pH was adjusted with concentrated NaOH solution.

Example 6
Dicopper edetate by basic copper carbonate-disodium edetate method

Ingredients:

100 ml water
2.39 g basic copper carbonate
3.72 g disodium edetate dihydrate

The disodium edetate was dissolved in the water. The basic copper carbonate was added slowly. The reaction was complete when no undissolved green material remained. In contrast to Examples 1—5, the chelate formed by the method of this example involved two copper ions per ligand molecule.

Example 7
Dicobaltous edetate by basic cobaltous carbonate-disodium edetate-sodium bicarbonate method

Ingredients:

50 ml water
2.07 g basic cobaltous carbonate, anhydrous
3.72 g disodium edetate, dihydrate trace sodium bicarbonate, anhydrous

The disodium edetate was dissolved in the water. The cobaltous carbonate was added slowly with stirring. Gradual evolution of bubbles occurred after enough of the metal salt has been added to raise the pH to roughly 5. At that point, the insoluble material began to disappear more rapidly. After all of the bubbling had stopped, the

liquid was stirred for approximately 15 minutes, and then the sodium bicarbonate was added slowly, with ample reaction time before further addition. The reaction was complete when a clear pink solution was obtained.

Example 8
Copper dithizone by basic copper carbonate-dithizone method

Ingredients:
5 ml water
20 ml chloroform
12.0 mg basic copper carbonate
25.6 mg dithizone

The dithizone was dissolved in the chloroform and the water was added. The basic copper carbonate was added and dispersed in the aqueous phase. The mixture was stirred well until no basic copper carbonate was visible in the aqueous phase when the phases were allowed to separate. This example also illustrates that the formation of the copper dithizone chelate can take place in non-aqueous media.

Example 9
Cupric caprylate by basic copper carbonate-caprylic acid-$CO_2$ gas method

Ingredients:
23.9 g basic copper carbonate (1.10 mole)
57.7 g caprylic acid (0.4 mole)
50 ml methanol
carbon dioxide gas

The basic copper carbonate was suspended in the methanol with vigorous stirring. The caprylic acid was added. The mixture was allowed to react with stirring. After the reaction appeared complete, the $CO_2$ was bubbled in very slowly until no further change took place. A greenish powder was isolated by washing the mixture with distilled water and vacuum drying.

Evolution of gas occurred during the initial stage of the reaction, with the formation of bubbles on the surface of the suspended salt. A definite color change took place. The pH immediately after the addition of the carboxylic acid was 4.6. Before the bubbling of the $CO_2$ into the mixture was begun, the pH was 5.7. While $CO_2$ was being bubbled into the mixture, the pH was 4.6. The reaction continued roughly 12 hours.

The isolated product was soluble (ca. 1 g/100 ml) in octanol. By comparison, basic copper carbonate is not soluble in octanol.

Example 10
Aluminum salicylate by sodium bicarbonate-salicylic acid method

Ingredients:
0.038 g $Al(OH)_3$ (dried into a soluble, friable powder by gentle heating)
0.20 g salicylic acid
0.002 g $NaHCO_3$
100 ml distilled water
NaOH solution

A. Salicylic acid was dissolved in the water. The aluminum hydroxide was added and suspended well. The $NaHCO_3$ was added, and the vessel was sealed immediately and agitated for 8 hours. The solution was adjusted to pH 8.0 for storage.

B. The above ingredients used in paragraph A were combined for another procedure in which the vessel was left open and the mixture stirred vigorously, immediately after addition of the $NaHCO_3$, to permit $CO_2$ gas to escape. The vessel was agitated for 8 hours and its contents adjusted to pH 8.0. This material was compared to the product of paragraph A. The material in the open vessel was a very cloudy white gelatinous suspension typical of aluminum hydroxide colloids. The material in the closed vessel was a low viscosity liquid, with a very slight haze. The hazy material was thought to be aluminum complex that had not finished reacting to form aluminum salicylate.

Example 11
Silver salicylate by sodium bicarbonate-salicylic acid method

Ingredients:
0.1 g AgOH, Hydrous
0.1 g Salicylic acid
50 ml $H_2O$
0.06 g $NaHCO_3$
NaOH solution

Salicylic acid was dissolved in water. The AgOH was ground into a fine powder and then added slowly to the solution with vigorous stirring. After all of the material was well suspended, the $NaHCO_3$ was added and the vessel was sealed immediately and placed in a dark area. The material in the vessel was agitated for 8 hours to produce silver salicylate.

Evaluation of the reactivity of copper hydroxide with citric acid in the presence and absence of bicarbonate ion

For the purpose of further illustrating the reaction occurring between $Cu(OH)_2$ and citric acid when basic copper carbonate is added to the acid, it was decided to isolate the $Cu(OH)_2$ moiety of basic copper carbonate and react this with citric acid in the presence and absence of sodium bicarbonate.

To prepare $Cu(OH)_2$ essentially free of $CuCO_3$, 0.1 mole of the mixed salt was reacted with excess HCl to bring the pH to 2.0. Gas evolved for about 10 minutes. The green supernatant solution was decanted and the green solid precipitate was collected, washed four times with distilled water (at ten times original volume) and collected for centrifugation. The solid was assumed to be $Cu(OH)_2 \cdot xH_2O$.

The reaction mixtures were prepared as follows, in separate culture tubes:

| Tube 1 | Tube 2 |
|---|---|
| 20 mM $Cu(OH)_2 \cdot xH_2O$ | 20 mM $Cu(OH)_2 \cdot xH_2O$ |
| 20 mM citric acid blank | 20 mM citric acid |
| 8 ml water | 1.2 mM $NaHCO_3$ 8 ml water |

The pH of the contents of Tube 2 was 2.6, and that of Tube 1 only 2.0. Therefore, NaOH solution was added to raise the pH of the contents of Tube 1 to 2.6. Both tubes were placed on a mechanical agitator for 8 hours with Tube 1 open and Tube 2 sealed to retain the sodium bicarbonate in the aqueous phase. At the end of this period, Tube 1 was sealed and Tube 2 allowed to stand overnight.

The following day, the pH of each mixture was adjusted to 8.0 with 4 normal NaOH solution, i.e., 3 ml were added to Tube 1 and 12 ml were added to Tube 2. The tubes were centrifuged to sediment any insoluble material. In Tube 1, a very pale blue supernatant liquid was found, with approximately 1.0 ml of pale green sediment, the color of the starting $CuOH_2$. In Tube 2, a dark blue supernatant (the color of the product formed from the reaction of citric acid and basic copper carbonate) was found, with about 0.2 ml of a green-black sediment.

Several conclusions were drawn from the above results. It was verified that the sediment in Tube 1 was unreacted $Cu(OH)_2 \cdot xH_2O$. The small amount of conversion of $Cu(OH)_2$ taking place in Tube 1 was due to contaminating $CuCO_3$ in the $Cu(OH)_2$ preparation. In Tube 2, 1:1 Cu:citrate complex was formed and verified. The green-black sediment found in Tube 2 was the product of the "activation" of $Cu(OH)_2$ caused by action of the $NaHCO_3$, and possibly the citric acid as well. If additional reaction time had been given the contents of Tube 2, the green-black material would have been converted into the 1:1 complex as has been verified by further experimental work.

The unique behavior of the gaseous $CO_2$ enables the otherwise relatively inert metal hydroxide to react. The results suggest catalytic action because the gas $CO_2$ participates in the reaction, but it is not consumed and may be recovered. A very unique aspect of this invention, thus, is the inclusion of the $CO_2$ gas that accelerates the reaction and yet is removable or even evolves during reaction. This activity is also considered unique and unexpected.

**Claims**

1. A method of preparing a salt or complex of an organic ligand which is a carboxylic acid or salt thereof and a metal selected from zinc, nickel, chromium, bismuth, mercury, silver, copper, magnesium, aluminium and cobalt, in which a hydroxide of such a metal is reacted with said organic ligand in an aqueous medium, characterised in that the reaction is conducted in the presence of an effective catalytic amount of gaseous $CO_2$.

2. A method as claimed in Claim 1, characterised in that the gaseous $CO_2$ is generated from the reaction mixture.

3. A method as claimed in Claim 1 or Claim 2, characterised in that the gaseous $CO_2$ is generated from carbonic acid, bicarbonate or carbonate present in the reaction mixture.

4. A method as claimed in Claim 3, characterised in that the reaction mixture comprises the carbonic acid salt of a metal as defined in claim 1 from which $CO_2$ is evolved.

5. A method as claimed in any preceding claim, characterised in that $CO_2$ is added during reaction.

6. A method as claimed in any preceding claim, characterised in that the reaction is conducted in a buffered acidic medium.

7. A method as claimed in any preceding claim, characterised in that the carboxylic acid is selected from hydroxypolycarboxylic, aminopolycarboxylic, sulphhydropolycarboxylic and phosphinolpolycarboxylic acid.

8. A method as claimed in any preceding claim, characterised in that the product is a dialkali metal monocopper (II) citrate.

9. A modification of the method claimed in Claim 1, characterised in that the organic ligand is dithizone, the $CO_2$ is generated from basic copper carbonate and the reaction is conducted in a 1:4 v/v mixture of water and chloroform.

10. A modification of the method claimed in Claim 1, characterised in that the organic ligand is caprylic acid and the reaction is conducted in the presence of basic copper carbonate and carbon dioxide in methanol without addition of water.

**Revendications**

1. Procédé de préparation d'un sel ou complexe d'un coordinat organique qui est un acide carboxylique ou un sel de celui-ci et un métal choisi parmi le zinz, le nickel, le chrome, le bismuth, le mercure, l'argent, le cuivre, le magnésium, l'aluminium et le cobalt dans lequel un hydroxyde d'un tel métal est mis en réaction avec ledit coordinat organique dans un milieu aqueux, caractérisé en ce que la réaction est conduite en présence d'une quantité catalytique utile de $CO_2$ gazeux.

2. Prcédé selon la revendication 1 caractérisé en ce que le $CO_2$ gazeux est produit par le mélange réactionnel.

3. Procédé selon l'une des revendications 1 ou 2 caractérisé en ce que le $CO_2$ gazeux est produit par l'acide carbonique, le bicarbonate ou le carbonate présent dans le mélange réactionnel.

4. Procédé selon la revendication 3 caractérisé en ce que le mélange réactionnel comprend l'acide carbonique sel d'un métal tel que défini dans la revendication 1 dont le $CO_2$ est dégagé.

5. Procédé selon l'une quelconque des précédentes revendications caractérisé en ce que le $CO_2$ es ajouté durant la réaction.

6. Procédé selon l'une quelconque des précédentes revendications caractérisé en ce que la réaction est conduite dans un milieu acide tamponé.

7. Procédé selon l'une quelconque des précédentes revendications caractérisé en ce que l'acide carboxylique est choisi parmi l'acide hydroxypolycarboxylique, l'acide aminopolycarboxylique, l'acide sulphhydropolycarboxylique et l'acide phosphinopolycarboxylique.

8. Procédé selon l'une quelconque des précédentes revendications, caractérisé en ce que le produit est un citrate bi-alcalin de métal monocuivre (II).

9. Variante du procédé revendiqué dans la revendication 1 caractérisé en ce que le coordinat organique est un dithizone dont le $CO_2$ est produit par un carbonate de cuivre basique et en ce que la réaction est conduite dans un mélange 1:4 v/v d'eau et de chloroforme.

10. Variante du procédé revendiqué dans la revendication 1 caractérisé en ce que le coordinat organique est un acide caprylique et en ce que la réaction est conduite en présence de carbonate de cuivre basique et de bioxyde de carbone dans le méthanol sans addition d'eau.

**Patentansprüche**

1. Ein Verfahren zur Herstellung eines Salzes oder Komplexes eines organischen Liganden, der eine Karbonsäure oder deren Salz ist, und eines Metalls, ausgewählt auz Zink, Nickel, Chrom, Wismut, Quecksilber, Silber, Kupfer, Magnesium, Aluminium und Kobalt, worin ein Hydroxid eines solchen Metalls mit dem besagten organischen Liganden in einem wässrigen Medium umgesetzt wird, dadurch gekennzeichnet, daß die Reaktion in Gegenwart einer wirksamen katalytischen Menge von gas förmigen $CO_2$ durchgeführt wird.

2. Ein Verfahren nach Anspruch 1, dadurch gekennzeichnet, daß das gasförmige $CO_2$ in der Reaktionmischung erzeugt wird.

3. Ein Verfahren nach Anspruch 1 oder 2, dadurch gekennzeichnet, daß das gasförmige $CO_2$ aus Kohlensäure, Bicarbonat oder Carbonat erzeugt wird, die in der Reaktionsmischung zugegen sind.

4. Ein Verfahren nach Anspruch 3, dadurch gekennzeichnet, daß die Reaktionsmischung das Kohlensäuresalz eines Metalls, wie es in Anspruch 1 definiert ist, enthält, aus dem $CO_2$ entwickelt wird.

5. Ein Verfahren nach irgendeinem der vorherigen Ansprüche, dadurch gekennzeichnet, daß $CO_2$ während der Reaktion zugegeben wird.

6. Ein Verfahren nach irgendeinem der vorherigen Ansprüche, dadurch gekennzeichnet, daß die Reaktion in einem gepufferten sauren Medium durchgeführt wird.

7. Ein Verfahren nach irgendeinem der vorherigen Ansprüche, dadurch gekennzeichnet, daß die Carbonsäure ausgewählt ist aus Hydroxidpolycarbonsäure, Aminopolycarbonsäure, Sulfhydropolycarbonsäure und Phosphinolpolycarbonsäure.

8. Ein Verfahren nach irgendeinem der vorigen Ansprüche, dadurch gekennzeichnet, daß das Produkt ein Dialkalimetallmonokupfer(II)Citrat ist.

9. Eine Modifikation des Verfahrens nach Anspruch 1, dadurch gekennzeichnet, daß der organische Ligand Dithizon ist, daß $CO_2$ aus Kupfercarbonat erzeugt wird und die Reaktion in einer 1:4 v/v Mischung von Wasser und Chloroform durchgeführt wird.

10. Eine Modifikation des Verfahrens nach Anspruch 1, dadurch gekennzeichnet, daß der organische Ligand Caprylsäure ist und die Reaktion in Gegenwart von basischem Kupfercarbonat und Kohlendioxid in Methanol ohne Zugabe von Wasser durchgeführt wird.